# EUROPEAN PATENT SPECIFICATION

(11) **EP 3 392 262 B1**
(45) Date of publication and mention of the grant of the patent: **18.08.2021**
(21) Application number: 18020140.2
(22) Date of filing: 11.04.2018
(51) Int. Cl.: C07K 5/12, A61K 38/12

(54) **CYCLOPEPTIDE, PHARMACEUTICAL OR COSMETIC COMPOSITION COMPRISING THE SAME AND METHOD FOR PREPARING THE SAME**
CYCLOPEPTID, ES ENTHALTENDE PHARMAZEUTISCHE ODER KOSMETISCHE ZUSAMMENSETZUNGEN UND SEIN HERSTELLUNGSVERFAHREN
CYCLOPEPTIDE ET COMPOSITIONS PHARMACEUTIQUES OU COSMÉTIQUES LE CONTENANT AINSI QUE PROCÉDÉ POUR SON PRÉPARATION

(30) Priority: 17.04.2017 US 201715488550; 05.03.2018 TW 107107206
(43) Date of publication of application: 24.10.2018
(60) Divisional application: 19207106.6
(73) Proprietor: National Tsing Hua University, Hsinchu City 30013 (TW)
(72) Inventor: Chen, Chien-Tien, 300 Hsinchu City (TW)
(74) Representative: Heyerhoff Geiger & Partner Patentanwälte PartGmbB

(56) References cited:
- WO-A1-2009/124754
- CN-B- 103 169 664
- DE-A1- 19 613 933
- KR-A- 20140 021 743

## Description

### BACKGROUND OF THE INVENTION

### 1. Field of the Invention

The present invention relates to a cyclopeptide, a pharmaceutical or cosmetic composition comprising the same and a method for preparing the same. More specifically, the present invention relates to a topical or cosmetic skin care cyclopeptide, a pharmaceutical or cosmetic composition comprising the same and a method for preparing the same.

### 2. Description of Related Art

Peptides have found widespread use in various fields, for example, topical or cosmetic skin care uses. Among the known peptides, the peptide with arginine (R)-glycine (G)-aspartate (D) motif is found as a common element in cellular recognition.

It is known that the peptide containing RGD motif can bind to the intergrin RGD binding site, and can be used to coat synthetic scaffolds in tissue engineering to enhance cellular attachment by mimicking *in vivo* WO 2009/124754 and DE 196 13 933 disclose cyclopeptides comprising the RGD motif and their use for topical pharmaceutical or cosmetic skin care.

In the conventional method for preparing the peptide containing RGD motif, coupling agents have to be used to catalyze the homogeneous or solid-phase peptide synthesis. However, the used amount of the coupling agents is not less, and the cost of the coupling agents itself is high. Hence, the production cost of the peptide is not low, and the obtained peptide cannot be available to all.

Therefore, it is desirable to provide a novel peptide containing RGD motif and a novel method for preparing the peptide; so, the obtained peptide can be widely applied to various fields.

### SUMMARY OF THE INVENTION

The object of the present invention is to provide a novel cyclopeptide and a pharmaceutical or cosmetic composition comprising the same, as defined by the appended claims 1 and 2.

### SUMMARY OF THE DISCLOSURE

RGD- and GRD-cyclopeptides of the present disclosure are respectively represented by the following formula (I) and (I'): 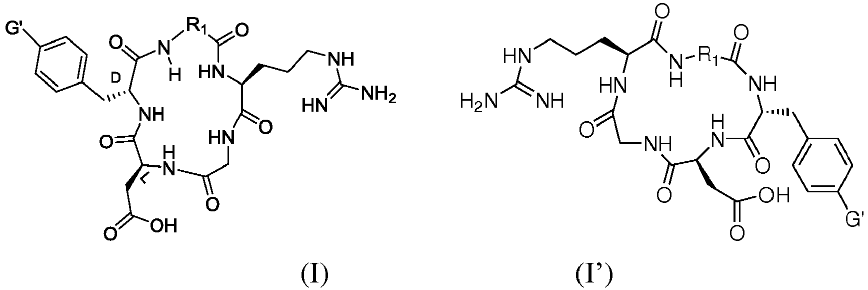 wherein,
R₁ is 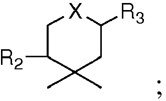 G' is H or OH;
each of R₂ and R₃ independently is H or C₁₋₆ alkyl;
X is O, S, CH₂ or N-R₄, in which R₄ is H, C₁₋₆ alkyl, (CH₂CH₂O)ₙH -C(=O)-C₁₋₁₀ alkyl, or C(=O)(C₂H₄)₂C(=O)O(C₂H₄O)ₙH, in which n = 1-3.

Preferably, in the cyclopeptide, R₃ is C₁₋₆ alkyl when X is CH₂.

The pharmaceutical or cosmetic composition comprises: an excipient, a Cu(II) ion or a VO(II) ion; and the aforementioned cyclopeptide.

In the cyclopeptide and the pharmaceutical or cosmetic composition X preferably is O, S, CH₂ or N-R₄, in which R₄ is H, C₁₋₆ alkyl, -C(=O)-C₄₋₁₀ alkyl, (CH₂CH₂O)ₙH, or C(=O)(C₂H₄)₂C(=O)O(C₂H₄O)ₙH, in which n = 1-3.

In the cyclopeptide and the pharmaceutical or cosmetic composition R₁ preferably is 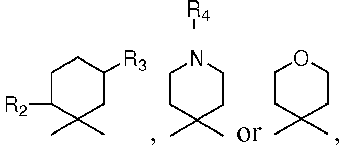 in which R₂ is H or C₁₋₆ alkyl, R₃ is C₁₋₆ alkyl; and R₄ is H, -C(=O)-C₄₋₁₀ alkyl or (CH₂CH₂O)ₙH. More preferably, R₂ is i-propyl, R₃ is methyl when R₁ is 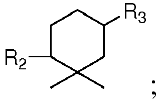 or R₄ is H or -C(=O)-heptyl when R₁ is

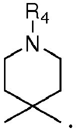

According to the present invention, the cyclopeptides of the present invention are represented by formulas (I-1) and (I-3):

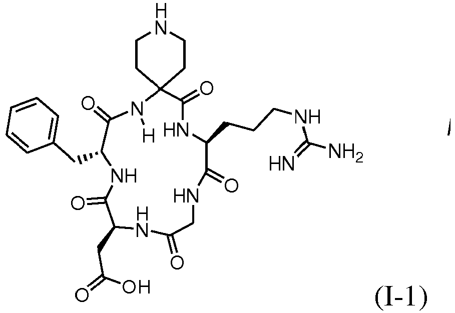

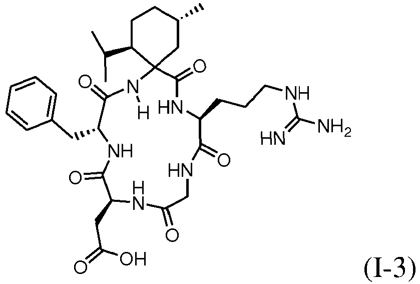

The cyclopeptide of the present invention comprises amino acids of arginine (R), glycine (G) and aspartate (D), which can bind to the intergrin RGD binding site. When the cyclopeptide of the present invention binds to the intergrin RGD binding site of the skin, the communication process between dermis and epidermis can be revived, and the production of important proteins of the basement membrane can be stimulated. Therefore, the purpose of ameliorating scars, wounds, inflammatory processes, aging and/or wrinkle formation can be achieved. Hence, the cyclopeptide and the pharmaceutical or cosmetic composition of the present invention can be applied to topical or cosmetic skin care composition.

In the pharmaceutical or cosmetic composition of the present invention, the suitable excipient for the present invention can be any excipient used in the art, for example, a binder, an anti-adhesive agent, a dispersant and a lubricant.

Except for the aforementioned cyclopeptide and pharmaceutical or cosmetic composition of the present invention, another object of the present invention is to provide a novel bio-compatible, catalytic method for preparing the cyclopeptide of the present invention.

The method of the present disclosure, which is not claimed, comprises the following steps (A) to (D).

In the step (A), compounds represented by the following formulas (II-1) or (II'-1), and (II-2) are provided from commercial source or made by our catalytic methods. 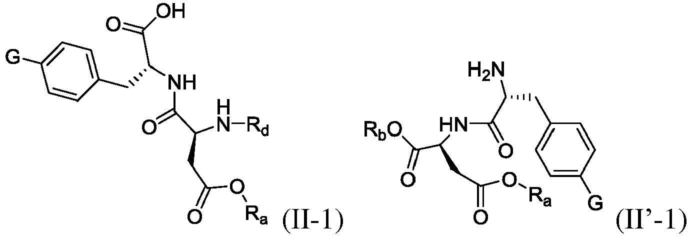 R_{c}-NH-R₁-COOH (II-2)
Herein, each of Rₐ and R_{b} independently is alkyl, cycloalkyl, aryl or heteroaryl;
R_{c} is a protection group;
G is H or OC(CH₃)₃; and
R₁ is 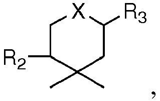 in which each of R₂ and R₃ independently is H or C₁₋₆ alkyl; X is O, S, CH₂ or N-R₄, in which R₄ is H, C₁₋₆ alkyl, (CH₂CH₂O)ₙH, -C(=O)-C₁₋₁₀ alkyl, or C(=O)(C₂H₄)₂C(=O)O(C₂H₄O)ₙH, in which n = 1-3.

In the step (B), a reaction between the compounds of formulas (II-1) or (II'-1) and (II-2) is performed to obtain a compound represented by the following formula (II-3) and (II'-3), respectively,

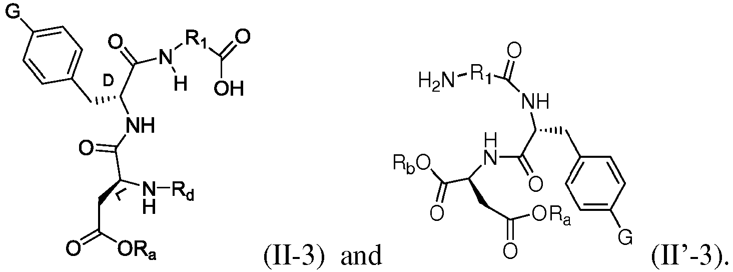

In the step (C), a reaction between the compound of formula (II-3) or (II'-3) and a compound represented by the following formula (II-4) or (II'-4), respectively, is performed to obtain a compound represented by the following formula (II-5) and (II'-5), respectively. 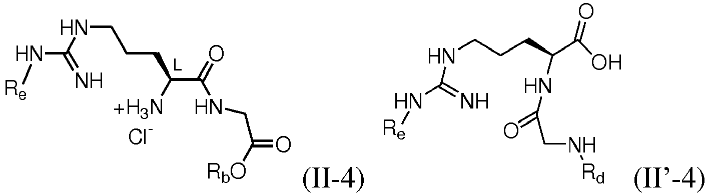 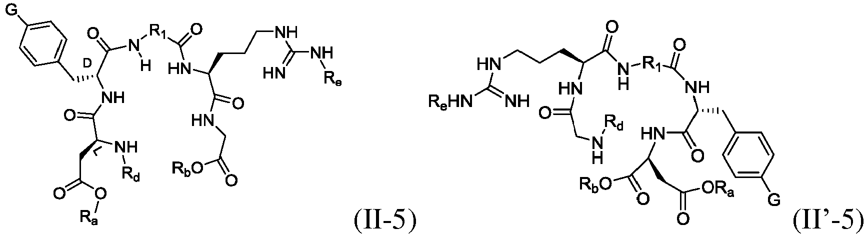 wherein, each of R_{d} and Rₑ independently is a protection group.

In the step (D), a cyclization reaction of the compound of formula (II-5) or (II'-5) is performed with a catalyst of formula (III), to obtain a compound represented by the formula (I) or (I'), respectively.

M(O)ₘL¹_{y}L²_{z} (III)

wherein M is a metal selected from the group consisting of IVB, VB, VIB and actinide groups;
L¹ and L² respectively is a ligand;
m and y are integers of greater than or equal to 1; and z is an integer of greater than or equal to zero.

In the method of the present invention, R_{c} and R_{d} can be Fluorenylmethyloxycarbonyl (Fmoc); and Rₑ can be MTr (4-methoxy-2,3,6-trimethylbenzenesulphonyl). However, the present invention is not limited thereto.

In the method of the present disclosure, the reaction between the compounds of formulas (II-1) or (II'-1) and (II-2) or the reaction between the compound of formula (II-3) and (II-4) or (II'-3) and (II'-4) can be performed with the catalyst of formula (III) or a coupling agent.

In the method of the present disclosure, when the reactions in the steps (B) to (D) are performed with the catalyst of formula (III), the catalyst used in the steps (B) to (D) can be the same or different.

In the catalyst of formula (III), L¹ is a ligand, which preferably is selected from the group consisting of Cl, OTf, OTs, NTf₂, halogen, RC(O)CHC(O)R, OAc, OC(O)CF₃, OEt, O-*i*Pr, and butyl, in which R is alkyl (preferably, C₁₋₆ alkyl; more preferably, C₁₋₃ alkyl). In addition, L² is also a ligand, which preferably is selected from the group consisting of Cl, H₂O, CH₃OH, EtOH, THF, CH₃CN and

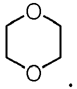

Furthermore, in the catalyst of formula (III), M can be a metal selected from the group consisting of IVB, VB, VIB and actinide groups. In one aspect, M is a group IVB transition element, m is 1 and y is 2; wherein M can be Ti, Zr of Hf. In another aspect, M is a group VB transition element, m is 1 and y is 2 or 3; wherein M can be V or Nb. In another aspect, M is a group VIB transition element, m is 1 and y is 4; wherein M is Mo, W or Cr. In another aspect, M is a group VIB transition element, m is 2 and y is 2; wherein M is Mo, W or Cr. In further another aspect, M is selected from the actinide group, m is 2 and y is 2; wherein M is U. Specific examples for the catalyst of formula (III) can be MoO₂Cl₂, V(O)OCl₂, V(O)(OAc)₂, V(O)(O₂CCF₃)₂, Ti(O)(acac)₂, Zr(O)Cl₂, Hf(O)Cl₂, Nb(O)Cl₂, MoO₂(acac)₂, V(O)(OTs)₂, V(O)(NTf₂)₂, or VO(OTf)₂, but the present disclosure is not limited thereto.

Furthermore, in the catalyst of formula (III), z can be an integer of greater than or equal to zero; and preferably, z is 0.

In the conventional method for preparing the cyclopeptide, 3-5 equivalent of coupling agents such as Hydroxybenzotriazole (HOBt), 1-Hydroxy-7-azabenzotriazole (HOAt), 2-(1H-benzotriazol-1-yl)-1,1,3,3-tetramethyluronium hexafluorophosphate (HBTU) and benzotriazol-1-yl-oxytripyrrolidinophosphonium hexafluorophosphate (PyBOP) are used. Because these coupling agents are expensive, the obtained cyclopeptide cannot be easily commercialized and applied to various fields.

In the method for preparing the cyclopeptide of the present invention, the catalyst of formula (III) is water soluble and used to facilitate the reaction progress. Hence, the expensive coupling agents are not used in the method of the present invention. Therefore, cyclopeptide can be produced in a cheaper manner, and the obtained cyclopeptide can be applied to various fields.

In the present disclosure alkyl, cycloalkyl, aryl, and heteroaryl present in the compounds include both substituted and unsubstituted moieties, unless specified otherwise. Possible substituents on alkyl, cycloalkyl, aryl, and heteroaryl include, but are not limited to, alkyl, alkenyl, halogen, alkoxy, ketone, alcohol, thioether, carbamate, amino, heterocyclic group or aryl; but alkyl cannot be substituted with alkyl.

In the present disclosure, the term "halogen" includes F, Cl, Br and I; and preferably is Cl or I. The term "alkyl" refers to linear and branched alkyl; preferably, includes linear and branched C₁₋₂₀ alkyl; more preferably, includes linear and branched C₁₋₁₂ alkyl; and most preferably, includes linear and branched C₁₋₆ alkyl. Specific examples of alkyl include, but are not limited to, methyl, ethyl, n-propyl, iso-propyl, n-butyl, sec-butyl, iso-butyl, tert-butyl, pentyl, neo-pentyl or hexyl. The term "alkoxy" refers to a moiety that the alkyl defined in the present invention coupled with an oxygen atom; preferably, includes linear and branched C₁₋₂₀ alkoxy; more preferably, includes linear and branched C₁₋₁₂ alkoxy; and most preferably, includes linear and branched C₁₋₆ alkoxy. Specific examples of alkoxy include, but are not limited to, methoxy, ethoxy, n-propoxy, iso-propoxy, n-butoxy, iso-butoxy, sec-butoxy, tert-butoxy, pentyloxy, neo-pentyloxy or hexyloxy. The term "alkenyl" refers to a linear or branched hydrocarbon moiety that contains at least one double bond; preferably, includes a linear and branched hydrocarbon C₂₋₂₀ moiety containing at least one double bond; more preferably, includes a linear and branched hydrocarbon C₂₋₁₂ moiety containing at least one double bond; and most preferably, includes a linear and branched hydrocarbon C₂₋₆ moiety containing at least one double bond. Specific examples of alkenyl include, but are not limited to, ethenyl, propenyl, allyl, or 1,4-butadienyl. The term "aryl" refers to a monovalent 6-carbon monocyclic, 10-carbon bicyclic, or 14-carbon tricyclic aromatic ring system. Specific examples of aryl include, but are not limited to, phenyl, naphthyl, pyrenyl, anthracenyl or phenanthryl; and preferably, the aryl is phenyl. The term "heterocyclic group" refers to a 5-8 membered monocyclic, 8-12 membered bicyclic or 11-14 membered tricyclic heteroaryl or heterocycloalkyl having at least one heteroatom which is selected from the group consisting of O, S and N. Specific examples of heterocyclic group include, but are not limited to, pyridyl, pyrimidinyl, furyl, thiazolyl, imidazolyl or thienyl.

### DETAILED DESCRIPTION OF THE PREFERRED EMBODIMENT

The cyclopeptide can be prepared as follows.

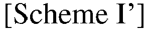

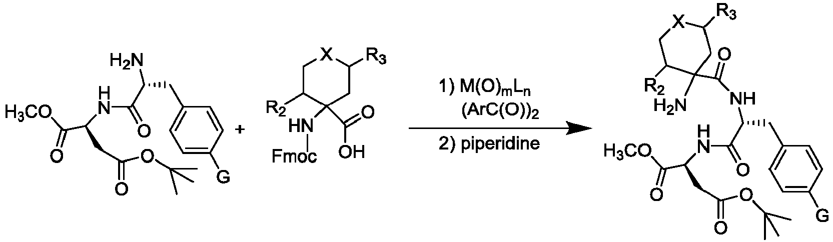

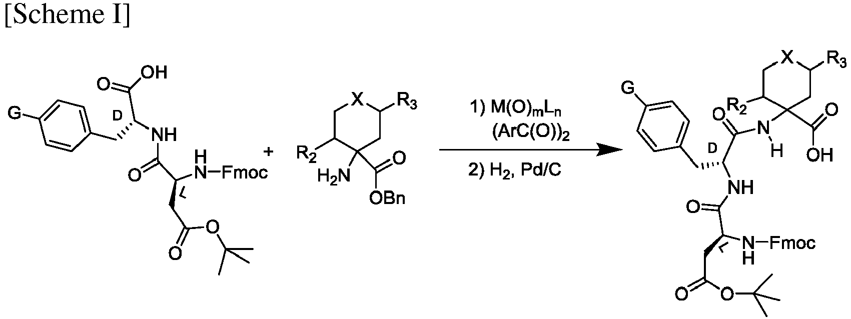

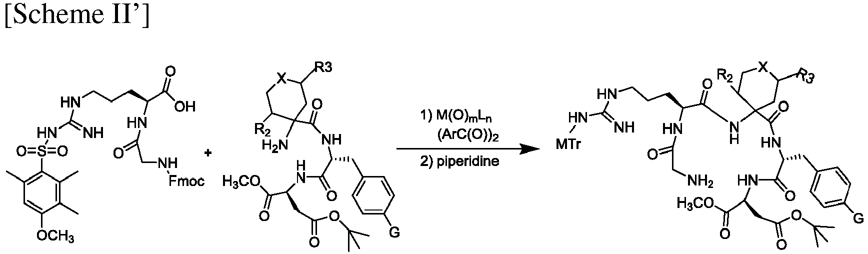

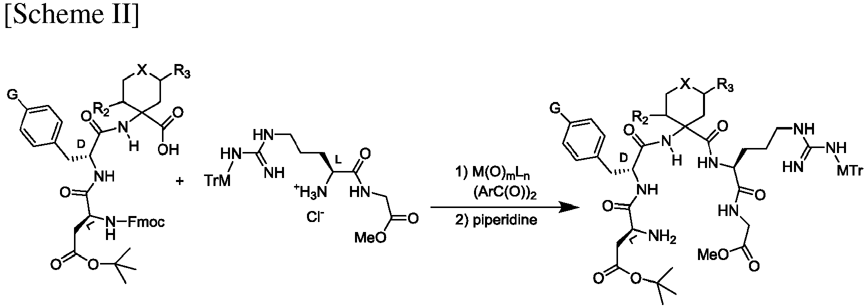

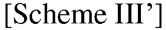

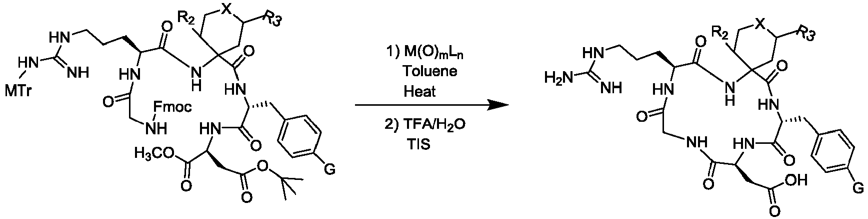

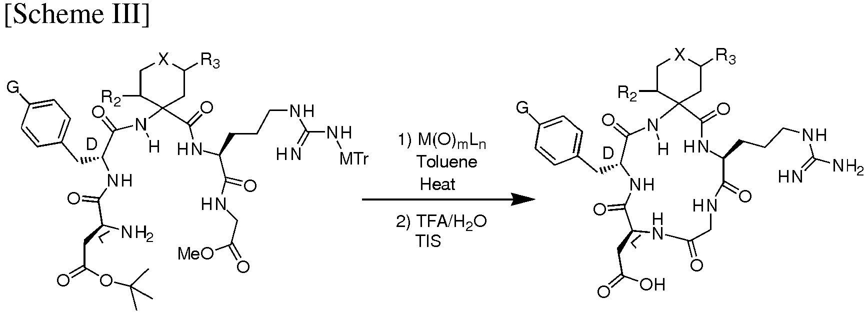

In Scheme I' and II', the coupling agents may also be used and can be, for example, Hydroxybenzotriazole (HOBt), 1-Hydroxy-7-azabenzotriazole (HOAt), 2-(1H-benzotriazol-1-yl)-1,1,3,3-tetramethyluronium hexafluorophosphate (HBTU) and benzotriazol-1-yl-oxytripyrrolidinophosphonium hexafluorophosphate (PyBOP).

Hereinafter, the present invention provides examples for preparing the cyclopeptide of the present invention; but the present is not limited thereto.

### 1-Amino-cis-4-methylcyclohexanecarboxylic Acid

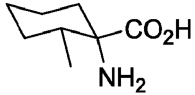

4-Methylcyclohexanone (45 g., 0.4 mole), potassium cyanide (30 *g.,* 0.4 mole), and ammonium chloride (22.0 g., 0.4 mole) were dissolved in water (300 ml.) and alcohol (250 ml.) and kept at room temperature for 6 days. The dark solution was diluted with water (300 ml.) and saturated with hydrogen chloride. After a further 2 days 1-amino-cis-4-methylcyclo-hexanenitrile hydrochloride (62-2 g., 88%) had crystallized. This hydrochloride (60 g.) was refluxed with 20% hydrochloric acid for 12 hr. The solution was evaporated to dryness, and the residue extracted (Soxhlet) with ethanol-ether (9 : 1) for 8 hr. After removal of the solvent, the residue was basified with aqueous ammonia, to yield 1-amino-cis-4-methylcyclo-hexanecarboxylic acid (45.5 g.), needles [from acetic acid-water (1 : 1)], m. p. 356-360' (sublimes), *R_{f}* 0.69 (C₈H₁₅NO₂, Found: C, 61.6; H, 9.7; N, 8.4%. Calc. for C₈H₁₅NO₂: C, 61.1; H, 9.6; N, 8.9%).

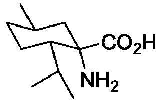

### trans-2-Isopropyl-5-methylcyclohexane-1-spiro-5'-hydantoin

Prepared in 37% yield from natural (-)-menthone, this spiran formed needles (from ethanol), m. p. 228-231.5° (Found: C, 63.0%; H, 8.8%; N, 11.6%.)

### 1-Amino-trans-2-isopropyl-5-methylcyclohexanecarboxylic acid

The previous hydantoin was hydrolysed by 60% sulphuric acid to the amino-acid, needles [from water-acetic acid (1:1)], m. p. 330 °C (C₁₁H₂₁NO₂, Found: C, 66.0; H, 10.5; N, 6.8 for C₁₁H₂₁NO₂: requires C, 66.3; H, 10.6; N, 7.0%).

### Dipeptide Fmoc-Asp(O^{t}Bu)-D-Phe-OH synthesis:

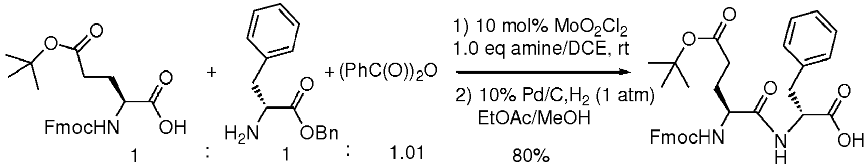

To a solution of Fmoc-Asp(O*^{t}*Bu)-OH (2.06 g, 5 mmol, 1.0 eq) in 1,2-dichloroethane (DCE, 10 mL) was added benzoic anhydride (1.14 g, 5.05 mmol, 1.01 eq) and MoO₂Cl₂ (100 mg, 0.5 mmol, 10 mol%) at room temperature under N₂ atmosphere and the reaction was monitored by TLC analysis. The reaction was stirred at room temperature for 2h till the starting amino acid was totally consumed and cooled to 0 °C. A solution of D-phenylalanine benzyl ester (1.275 g, 5.0 mmol, 1.0 eq) in 5 mL of DCE was added to the above solution via syringe follow by the addition of amine base (5.0 mmol, 1.0 eq) at 0 °C. The reaction mixture was allowed stir at room temperature for 30 min. Solvent was evaporated, and the remaining residue was dissolved in EtOAc (100 mL), washed with saturated aqueous NaHCO₃ (30 mL), H₂O (30 mL), brine (30 mL), and dried over Na₂SO₄. After evaporation of solvent, the remaining residue was purified by flash chromatography on silica gel to provide Fmoc-Asp(O*^{t}*Bu)-D-Phe-OBn (2.68 g, 81% yield) as a white solid: TLC *R_{f}*= 0.5 (EtOAc/Hexane=1/5); ¹H NMR (400 MHz, CDCl₃): δ 7.77 (d, *J* = 7.6, 2H), 7.57 (q, *J* = 3.6, 2H), 7.41 (t, *J* = 7.6, 2H), 7.35-7.32 (m, 4H), 7.30-7.27 (m, 4H), 7.17 (t, *J* = 6.0, 3H), 7.02 (t, *J* = 6.4, 3H), 5.83 (br, 1H), 5.13 (q, *J* = 12.0, 2H), 4.87 (q, *J* = 7.2, 1H), 4.58-4.49 (m, 1H), 4.36 (d, *J* = 7.2, 2H), 4.20 (t, *J* = 7.2, 1H), 3.10 (dd, *J* = 16.4, 5.6, 2H), 1.43 (s, 9H); ¹³C NMR (100 MHz, CDCl₃) δ 170.8, 170.0, 156.0, 143.8, 143.6, 141.3, 135.5, 135.0, 129.2, 128.6, 128.5, 127.7, 127.1, 125.1, 120.0, 81.9, 67.3, 67.2, 53.4, 51.1, 47.0, 37.8, 37.3, 28.0; HRMS (ESI), Calcd. for C₄₀H₄₂N₂NaO₇ ([M+Na]⁺): 685.2889, found: 685.2887.

To a solution of Fmoc-Asp(O*^{t}*Bu)-D-Phe-OBn (2.0 g, 3.0 mmol, 1 equiv) in 150 mL of 1/1(v/v) ratio of EtOAc/MeOH was added 10% Pd/C (383 mg, 10 mol %) at RT. The reaction was allowed to stir in an atmosphere of hydrogen (balloon) over 1.5 h, following which it was filtered over Celite. The Celite was washed multiple times with MeOH (30 mL), EtOAc (30 mL) and the combined filtrate was concentrated in vacuo to give 1.699 g (99%) of dipeptide Fmoc-Asp(O*^{t}*Bu)-D-Phe-OH as a white solid. TLC R*_{f}*= 0.23 (EtOAc/Hex=2/1)

¹H NMR (300 MHz, CDCl₃): δ 7.75 (d, *J* = 7.5, 2H), 7.54 (d, *J* = 7.2, 2H), 7.39 (t, *J* = 7.5, 2H), 7.29 (d, *J* = 7.2, 2H), 7.23-7.16 (m, 5H), 7.06 (d, *J* = 7.5, 1H), 6.12 (d, *J* = 8.7, 1H), 3.21 (dd, *J* = 9.6, 6.3, 1H), 3.06 (dd, *J* = 9.6, 6.6, 1H), 2.72 (dd, *J* = 16.8, 6.3, 1H), 2.57 (dd, *J* = 16.2, 5.7, 1H), 1.43 (s, 9H); ¹³C NMR (100 MHz, CDCl₃) δ 171.4, 170.8, 170.0, 156.0, 143.7, 143.6, 141.2, 135.7, 129.2, 128.5, 127.7, 127.1, 125.1, 120.0, 81.9, 67.3, 53.4, 52.3, 51.0, 47.0, 37.9, 37.4, 29.7, 28.0; HRMS (ESI), Calcd. for C₃₃H₃₆N₂NaO₇ ([M+Na]⁺): 595.2420, found: 595.2423.

### Dipeptide Fmoc-Arg(Mtr)-Gly-OCH₃ synthesis by EDC-HOBt coupling

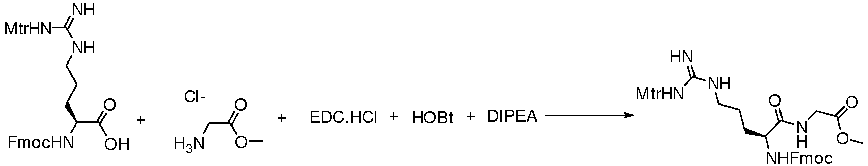

¹H NMR (400 MHz, CDCl₃): δ 7.72 (d, *J* = 7.6, 2H), 7.63-7.54 (m, 3H), 7.25 (t, *J* = 7.6, 2H), 7.24-7.22 (m, 1H), 6.49 (br, 1H), 6.40 (br, 1H), 6.07 (d, *J* = 8.0 1H), 4.32 (q, *J* = 6.8, 3H), 4.14 (t, *J* = 7.2, 1H), 4.02 (dd, *J* = 17.6, 5.2, 1H), 3.89 (dd, *J* = 17.6, 5.2, 1H), 3.78 (s, 3H), 3.66 (s, 3H), 3.34-3.23 (m, 2H), 2.66 (s, 3H), 2.60 (s, 3H), 2.16 (s, 3H), 2.05 (s, 3H), 1.93 (t, *J* = 6.0, 1H), 1.72-1.60 (m, 3H), 1.27 (t, *J* = 6.8, 1H); ¹³C NMR (100 MHz, CDCl₃) 172.8, 170.6, 158.6, 156.5, 143.8, 143.7, 141.2, 138.5, 136.6, 134.7, 134.1, 133.0, 129.1, 127.7, 125.1, 124.9, 124.3, 120.3, 120.0, 111.7, 67.1, 60.4, 55.4, 52.3, 47.0, 41.0, 40.2, 31.9, 30.0, 29.7, 25.1, 24.0, 21.0, 18.3; HRMS (ESI), Calcd. for C₃₄H₄₁N₅NaO₈S ([M+Na]⁺): 702.2573, found: 702.2575.

### Example 1

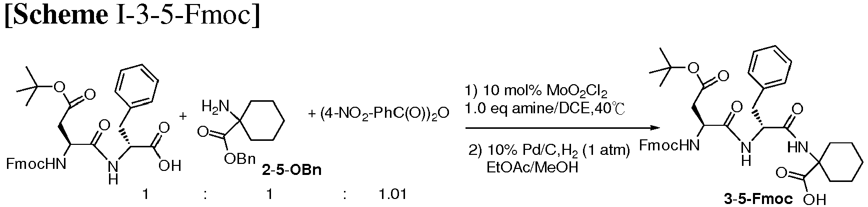

To a solution of Fmoc-Asp(O*^{t}*Bu)-D-Phe-OH (1.6 g, 2.8 mmol, 1.0 eq) in 1,2-dichloroethane (DCE, 5 mL) was added 2,6-dinitrobenzoic anhydride (974 mg, 2.82 mmol, 1.01 eq) and MoO₂Cl₂ (56 mg, 0.28 mmol, 10 mol%) at room temperature and gradually heated 40 °C under N₂ atmosphere and the reaction was monitored by TLC analysis. The reaction was stirred at 40 °C for 2h till the starting amino acid was totally consumed and cooled to 0 °C. A solution of 1-aminocyclohexanecarboxylic acid benzyl ester (653 mg, 2.8 mmol) in 3 mL DCE was added to the above solution via syringe follow by the addition of amine (2.8 mmol, 1.0 eq) at 0 °C. The reaction mixture was allowed stir at room temperature for 12 h. Solvent was evaporated, and the remaining residue was dissolved in EtOAc, washed with saturated aqueous NaHCO₃ (1 mL), H₂O (1 mL), brine (1 mL), and dried over Na₂SO₄. After evaporation of solvent, the remaining residue was purified by silica gel flash chromatography to provide Fmoc-Asp(O*^{t}*Bu)-D-Phe-ACHA-OBn (1.69 g, 77% yield) as a white solid: TLC R*_{f}*= 0.26 (EtOAc/Hexane=1/5); ¹H NMR (400 MHz, CDCl₃): δ 7.77 (d, *J* = 7.6, 2H), 7.57 (d, *J* = 7.2, 2H), 7.41 (t, *J* = 7.6, 2H), 7.35-7.27 (m, 7H), 7.24-7.16 (m, 5H), 6.95-7.89 (br, 1H), 6.21 (br, 1H), 5.72 (br, 1H), 5.11 (q, *J* = 8.0, 2H), 4.63 (q, *J* = 7.6, 1H), 4.43-4.21 (m, 3H), 4.23 (t, *J* = 7.2, 1H), 3.07 (dd, *J* = 16.4, 6.4, 1H), 2.98-2.92 (m, 1H), 2.81 (dd, *J* = 16.2,4.8, 1H), 2.64-2.53 (m, 1H), 1.85-1.79 (m, 4H), 1.64-1.49 (m, 3H), 1.47 (s, 9H), 1.26-1.20 (m, 4H); ¹³C NMR (100 MHz, CDCl₃) δ 173.5, 170.8, 170.5, 169.6, 143.6, 141.3, 136.0, 129.4, 128.7, 128.5, 128.2, 127.8, 127.1, 127.0, 125.0, 120.0, 82.0, 67.3, 66.8, 59.0, 54.1, 51.4, 47.1, 37.3, 32.3, 32.1, 28.0, 24.9, 21.2, 18.3; HRMS (ESI), Calcd. for C₄₆H₅₁N₃NaO₈ ([M+Na]⁺): 796.3574, found: 796.3580.

To a solution of Fmoc-Asp(O*^{t}*Bu)-D-Phe-ACHA-OBn (1.5 g, 1.9 mmol, 1 equiv) in 100 mL of 1/1(v/v) ratio of EtOAc/MeOH was added 10% Pd/C (242 mg, 10 mol %) at RT. The reaction was allowed to stir in an atmosphere of hydrogen (balloon) over 2h, following which it was filtered over Celite. The Celite was washed multiple times with MeOH (20 mL), EtOAc (20 mL) and the combined filtrate was concentrated in vacuo to give 1.31 g (98%) of dipeptide Fmoc-Asp(O*^{t}*Bu)-D-Phe-ACHA-OH as a white solid (**3-5-Fmoc**): TLC R*_{f}* = 0.24 (EtOAc/Hex=2/1); ¹H NMR (400 MHz, CDCl₃): δ 7.75 (d, *J* = 7.2, 2H), 7.73-7.72 (m, 6H), 7.40-7.32 (m, 5H), 7.31-7.21 (m, 6H), 6.83 (d, *J* = 16.0, 1H), 6.46 (d, *J* = 5.4, 1H), 6.03 (br, 1H), 4.73 (q, *J* = 7.6, 1H), 4.48-4.42 (m, 1H), 4.41-4.12 (m, 4H), 3.19-3.02 (m, 2H), 2.84-2.68 (m, 2H), 2.18-1.65 (m, 4H), 1.49 (s, 9H), 1.63-1.24 (m, 6 H); ¹³C NMR (100 MHz, CDCl₃) δ 176.6, 171.6, 171.3, 171.1, 170.7, 156.2, 143.7, 141.2, 136.6, 136.5, 129.3, 128.6, 127.7, 127.1, 126.9, 125.1, 120.0, 81.7, 67.5, 59.4, 54.7, 52.1, 51.6, 47.0, 38.0, 37.3, 36.9, 36.7, 32.9, 31.0, 29.7, 28.0, 25.0, 21.3, 21.1; HRMS (ESI), Calcd. for C₃₉H₄₅N₃NaO₈ ([M+Na]⁺): 706.3104, found: 706.3108.

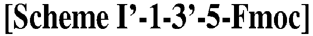

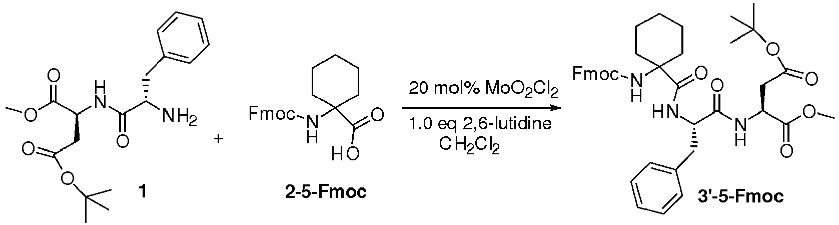

Step A: A solution of H-D-Phe-Asp(OtBu)-OMe.HCl (1-HCl) (48.7 mg, 0.126 mmol, 1.01 eq) in MeOH (1 mL) was cooled to 0°C, 1.5 eq NaHCO₃ was added and stirred for 0.5 h at rt. MeOH was evaporated and the resulting residual was dissolved in 2 mL THF and dried with Na₂SO₄, and filtered. The solvent was removed and dried under vacuum to obtain D-Phe-Asp(OtBu)-OMe (1).

In a dry 50-mL, two-necked, round-bottomed flask was charged with MoO₂Cl₂ (5.0 mg, 0.025mmol, 20 mol%) in anhydrous CH₂Cl₂(1.0 mL). To the above solution, Fmoc-1-aminocyclohexane-1-carboxylic acid (**2-5-Fmoc**) (48.3 mg, 0.125 mmol) was added at ambient temperature followed by addition of benzoic anhydride (29 mg, 0.127 mmol), and heated at 40 °C for 6 h then cooled to 0 °C.

A solution of D-Phe-Asp(OtBu)-OMe (48.3 mg, 0.125 mmol) in DCM (0.5 mL) was added to above solution at 0 °C and gradually raise temperature to rt and stir at rt for 2 h. Afterward 14µL of 2,6-lutidine was added and continued stirred for additional 4h at rt. The reaction was quenched with water (2mL) and the organic phase was separated and the aqueous phase was extracted with dichloromethane (10mLx2). The combined organic phase were dried over anhydrous Na₂SO₄ and concentrated. The crude product was purified by column chromatography on silica gel (EA/Hex=2/3) and gave Fmoc-protected tripeptide **(3'-5-Fmoc)** (58mg, 67%).

The procedure for preparing the tripeptide (**3'**) is not limited to the above procedure, and Fmoc-protected tripeptide (**3'-5-Fmoc**) can be prepared, for example, by the following Scheme I'-3'-5-Fmoc-B.

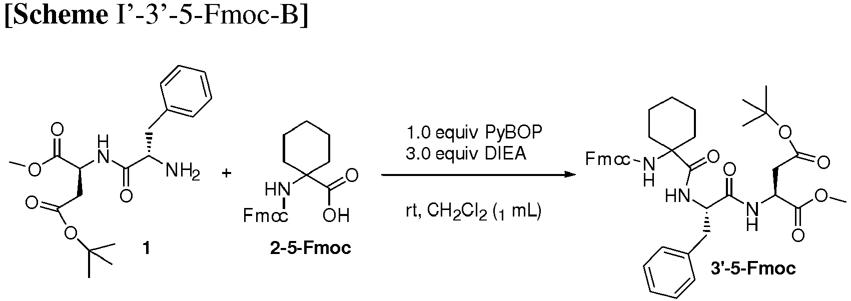

Step B: In a dry 25-mL, two-necked, round-bottomed flask was charged with coupling reagent (1.0 equiv) in DCM (1 mL/mmol) and treated under stirring with DIEA (3.0 equiv) at 0 °C for 5 min. Fmoc-1-aminocyclohexane-1-carboxylic acid (**2-5-Fmoc**) (182.7 mg, 0.5 mmol) was added at 0 °C for 10 min, and mixed with H-D-Phe-Asp(OtBu)-OMe.HCl (1) (192.7 mg, 0.55 mmol). The ice bath was removed after 10 min and the stirring continued at room temperature 96 h. The mixture was poured into AcOEt (50 x the DCM volume) and the solution treated according to the usual workup. The crude product was purified by column chromatography on silica gel and gave Fmoc-protected tripeptide (**3'-5-Fmoc**)**.**Yield: 68% Purified by column chromatography (EtOAc/hexanes = 3:7, R*_{f}*=0.2) ¹H NMR (400 MHz, CDCl₃) δ 7.74 (d, *J* = 7.2 Hz, 2H), 7.57 (d, *J* = 7.2 Hz, 2H), 7.40-7.27 (m, 4H), 7.19-7.07 (m, 5H), 6.63 (d, *J* = 8.0 Hz, 1H), 5.11 (s, 1H), 4.84 (d, *J* = 6.6 Hz, 1H), 4.74 (d, *J* = 5.4 Hz, 1H), 4.41-4.32 (m, 2H), 4.15 (t, *J* = 6.4 Hz, 1H), 3.59 (s, 3H), 3.23 (dd, *J* = 14.0, 6.9 Hz, 1H), 3.04 (dd, *J* = 14.0, 8.2 Hz, 1H), 2.76-2.63 (m, 2H), 1.92-1.77 (m, 2H), 1.60-1.47 (m, 5H), 1.38 (s, 9H), 1.27-1.18 (m, 3H); ¹³C NMR (100 MHz, CDCl₃) δ 173.8, 171.2, 170.6, 169.2, 155.3, 143.6, 143.5, 141.1, 136.6, 128.9, 128.3, 127.6, 126.9, 126.9, 124.8, 124.8, 119.8, 119.8, 81.3, 66.7, 59.3, 53.5, 52.1, 48.7, 47.0, 37.2, 37.0, 32.4, 30.9, 27.7, 24.8, 21.1, 21.0; HRMS (ESI) calcd for C₄₀H₄₇N₃O₈ (M+⁺Na): 720.3255; found: 720.3253.

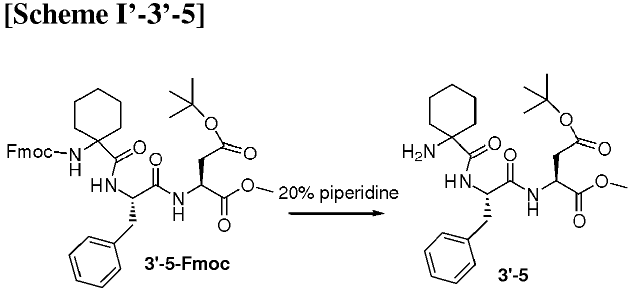

A solution of 200 mg (0.28 mmol) of the Fmoc-protected tripeptide (**3'-5-Fmoc**) was treated with 20% piperidine in DCM (1 mL) for 1 hour at room temperature. After removal of piperidine by coevaporation with methanol, the crude product was dried in vacuo and purified by column chromatography on silica gel to obtain tripeptide (**3'-5**)**.**
Yield: 106.8/136.2 = 78 %
Purified by column chromatography (EtOAc/hexanes = 9:1, R*_{f}* =0.2) ¹H NMR (400 MHz, CDCl₃) δ 8.21 (d, *J* = 8.2 Hz, 1H, NH), 7.21 (t, *J* = 6.8 Hz, 2H), 7.16-7.14 (m, 3H), 7.05 (d, *J* = 8.4 Hz, 1H), 4.76-4.72 (m, 1H), 4.63-4.58 (m, 1H), 3.66 (s, 3H, OCH₃), 3.15 (dd, *J* = 14.0, 6.2 Hz, 1H), 3.00 (dd, *J* = 14.0, 8.0 Hz, 1H), 2.81 (dd, *J* = 16.0, 4.0 Hz, 1H), 2.51 (dd, *J* = 16.0, 4.0 Hz, 1H), 1.97-1.72 (m, 2H), 1.58-1.51 (m, 4H), 1.35 (s, 9H, C(CH₃)₃), 1.36-1.28 (m, 2H), 1.24-1.09 (m, 2H); ¹³C NMR (100 MHz, CDCl₃) δ 178.3, 170.9, 170.8, 169.9, 136.8, 129.1, 128.3, 126.6, 81.7, 57.1, 53.8, 52.4, 48.2, 37.5, 37.1, 34.4, 34.1, 27.0, 25.0, 21.0; HRMS (ESI) calcd for C₂₅H₃₇N₃O₆ (M+H⁺): 476.2761; found: 476.2753.

### Example 2

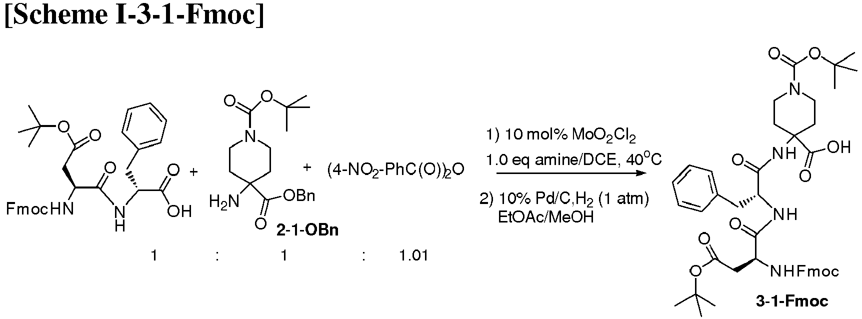

Purified by column chromatography (EtOAc/hexanes = 3:1, R*_{f}* =0.32); ¹H NMR (400 MHz, CDCl₃): δ 7.74 (d, *J* = 7.4, 2H), 7.75-7.70 (m, 4H), 7.38-7.30 (m, 2H), 7.31-7.21 7.19-7.14 (m, 5H), 6.85 (d, *J* = 16.0, 1H, NH), 6.48 (d, *J* = 5.4, 1H, NH), 6.35 (br, 1H, NH), 4.75 (q, *J* = 7.4, 1H), 4.52-4.45 (m, 1H), 4.42-4.13 (m, 3H), 3.78-3.73 (m, 2H), 3.37-3.33 (m, 2H), 3.22-3.06 (m, 2H), 2.85-2.67 (m, 2H), 2.38-2.23 (m, 2H), 2.05-1.83 (m, 2H), 1.49 (s, 9H), 1.42 (s, 9H); HRMS (ESI), Calcd. for C₄₃H₅₂N₄NaO₁₀([M+Na]⁺): 807.3581, found: 807.3577.

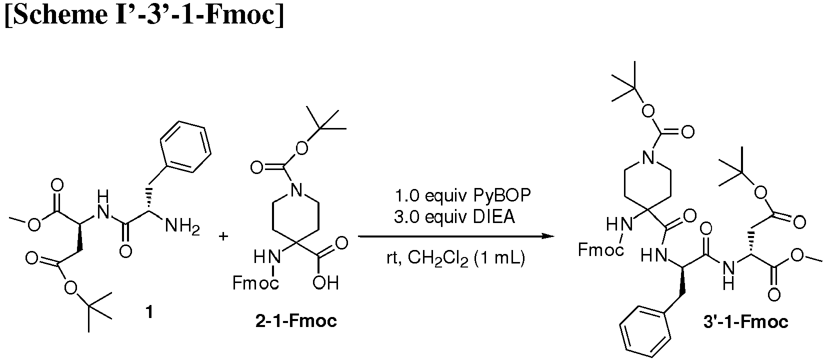

In a dry 25-mL, two-necked, round-bottomed flask was charged with coupling reagent (1.0 equiv) in DCM (1 mL/mmol) and treated under stirring with DIEA (3.0 equiv) at 0 °C for 5 min. 1-Boc-piperidine-4-Fmoc-amino-1-carboxylic acid (**2-1-Fmoc**) (233.27 mg, 0.5 mmol) was added at 0 °C for 10 min, and mixed with H-D-Phe-Asp(OtBu)-OMe.HCl (1) (202.91 mg, 0.525 mmol). The ice bath was removed after 10 min and the stirring continued at room temperature 96 h. The mixture was poured into AcOEt (50 x the DCM volume) and the solution treated according to the usual workup. The crude product was purified by column chromatography on silica gel and gave Fmoc-protected tripeptide (**3'-1-Fmoc**).
Yield: 259/399.17 = 65 %
Purified by column chromatography (EtOAc/hexanes = 3:7, R*_{f}* =0.2) ¹H NMR (400 MHz, CDCl₃) δ 7.75 (d, *J* = 7.6 Hz, 2H), 7.56 (t, *J* = 5.2 Hz, 2H), 7.39 (dt, *J* = 6.4, 2.4 Hz, 2H), 7.32-7.27 (m, 2H), 7.19 (t, *J* = 7.6 Hz, 2H), 7.12 (t, *J* = 6.2 Hz, 3H), 6.68 (d, *J* = 7.6 Hz, 1H), 4.79 (s, 1H), 4.44 (d, *J* = 6.0 Hz, 1H), 4.15 (t, *J* = 6.2 Hz, 1H), 3.74-3.67 (m, 1H), 3.63 (s, 3H), 3.56 (d, *J* = 14.0 Hz, 1H), 3.19 (br, 1H), 2.99 (dd, *J* = 14.0, 8.2 Hz, 1H), 2.89 (br, 1H), 2.76 (dd, *J* = 16.0, 6.0 Hz, 1H), 2.60 (d, *J* = 12.8 Hz, 1H), 2.05-1.96 (m, 1H), 1.77-1.53 (m, 6H), 1.42 (s, 9H), 1.38 (s, 9H); ¹³C NMR (100 MHz, CDCl₃) δ 172.7, 171.2, 170.5, 169.3, 155.3, 154.3, 143.5, 143.4, 141.1, 136.5, 128.9, 128.3, 127.6, 126.9, 126.9, 126.7, 124.8, 124.7, 119.8, 81.4, 79.6, 66.7, 57.6, 53.6, 52.3, 48.7, 46.9, 37.1, 28.2, 27.8.

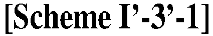

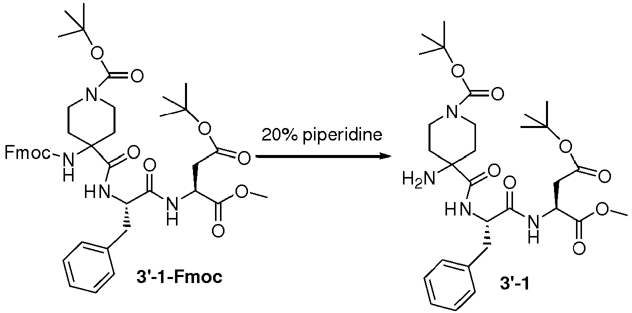

A solution of 219 mg (0.27 mmol) of the Fmoc-protected tripeptide (**3'-1-Fmoc**) was treated with 20% piperidine in DCM (1 mL) for 3 hour at room temperature. After removal of piperidine by coevaporation with methanol, the crude product was dried in vacuo and purified by column chromatography on silica gel to obtain tripeptide (**3'-1**).
Yield: 113/155.6 = 72 %
Purified by column chromatography (EtOAc/hexanes = 9:1, R*_{f}* =0.2) ¹H NMR (400 MHz, CDCl₃) δ 8.05 (d, *J* = 8.2 Hz, 1H, NH), 7.26-7.14 (m, 5H), 7.05 (d, *J* = 8.4 Hz, 1H), 4.76-4.72 (m, 1H), 4.66-4.60 (m, 1H), 3.88-3.76 (m, 2H, NH₂), 3.67 (s, 3H, OCH₃), 3.16 (dd, *J* = 14.0, 6.2 Hz, 1H), 3.02-2.92 (m, 3H), 2.82 (dd, *J* = 17.0, 4.4 Hz, 1H), 2.52 (dd, *J* = 17.0, 4.6 Hz, 1H), 2.07-2.00 (m, 1H), 1.91-1.84 (m, 1H), 1.58-1.38 (m, 1H), 1.40 (s, 9H, C(CH₃)₃), 1.36 (s, 9H, C(CH₃)₃), 1.32-1.20 (m, 2H), 1.11-1.06 (m, 1H); ¹³C NMR (100 MHz, CDCl₃) δ 176.7, 170.9, 170.6, 169.9, 154.4, 136.6, 129.1, 128.4, 126.7, 81.7, 79.4, 55.3, 53.7, 52.4, 48.2, 37.5, 37.0, 34.2, 28.2, 27.8; HRMS (ESI) calcd for C₂₉H₄₅N₄O₈ (M⁺+1): 577.3232; found: 577.3248.

### Example 3

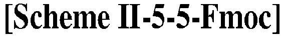

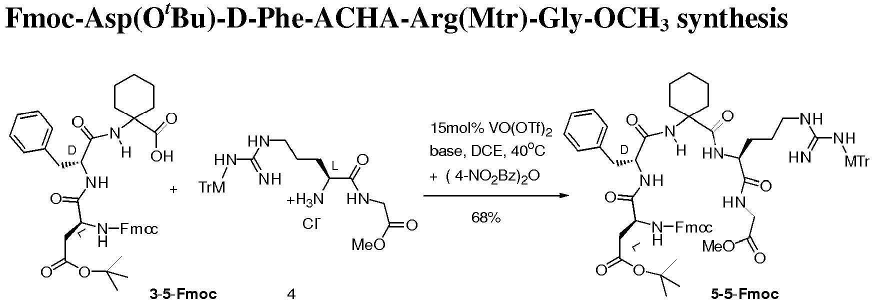

To a solution of Fmoc-Asp(O*^{t}*Bu)-D-Phe-ACHA-OH (**3-5-Fmoc**) (698 g, 1 mmol, 1.0 eq) in 1,2-dichloroethane (DCE, 5 mL) was added 2,6-dinitrobenzoic anhydride (348 mg, 1.01 mmol, 1.01 eq) and VO(OTf)₂ (55 mg, 0.15 mmol, 15 mol%) at room temperature and gradually heated 40 °C under N₂ atmosphere and the reaction was monitored by TLC analysis. The reaction was stirred at 40 °C for 4h till the starting amino acid was totally consumed and cooled to 0 °C. A solution of NH₂-Arg(Mtr)-Gly-OCH₃ (**4**) (457 mg, 1 mmol) in 3 mL DCE was added to the above solution via syringe follow by the addition of base (1.0 mmol, 1.0 eq) at 0 °C. The reaction mixture was allowed stir at room temperature for 12 h. Solvent was evaporated, and the remaining residue was dissolved in EtOAc (100 mL), washed with saturated aqueous NaHCO₃ (20 mL), H₂O (20 mL), brine (20 mL), and dried over Na₂SO₄. After evaporation of solvent, the remaining residue was purified by flash chromatography on silica gel to provide Fmoc-Asp(O*^{t}*Bu)-D-Phe-ACHA-Arg(Mtr)-Gly-OCH₃ (**5-5-Fmoc**) (612 mg, 68% yield) as a white solid: TLC R*_{f}*=0.50 (EtOAc/Hexane=3/1); ¹H NMR (400 MHz, CDCl₃): δ 7.75 (d, 2 H, *J*=7.20 Hz), 7.57 (m, 2 H), 7.39 (t, 2 H, *J*=7.6 Hz), 7.30-7.18 (m, 8H), 6.60 (br, 1 H), 6.55 (br, 1 H), 4.67 (br, 1 H), 4.52-4.42 (m, 1H), 4.37 (q, 1 H, *J*=6.40 Hz), 4.35-4.23 (m, 2H), 4.19 (t, 1 H, *J*=7.2 Hz), 4.14-4.04 (m, 1H), 3.81 (s, 3H, OCH₃), 3.69-3.61 (m, 1H), 3.58 (s, 3H, CO₂CH₃), 3.46-3.42 (m, 1H), 3.21-3.09 (m, 2H), 3.03-2.92 (m, 1H), 2.68 (s, 3H), 2.59 (s, 3H), 2.25-2.11 (m, 2H), 2.10 (s, 3H), 2.04-1.83 (m, 4H), 1.79-1.58 (m, 3H), 1.59-1.41 (m, 4H), 1.39 (s, 9 H, C(CH₃)₃), 1.35-1.05 (m, 4H); ¹³C NMR (100 MHz, CDCl₃) δ175.4, 172.9, 172.6, 172.0, 171.2, 170.5, 156.7, 143.6, 141.2, 136.3, 129.2, 129.0, 128.7, 127.8, 127.1, 127.0, 125.0, 120.0, 112.3, 81.7, 77.3, 67.2, 60.4, 55.5, 53.0. 52.3, 51.2, 47.0, 41.2, 37.0, 36.7, 34.1, 30.0, 29.0, 28.0, 24.9, 24.3, 21.3, 20.9, 18.3, 12.0; HRMS (ESI), calculated for C₅₈H₇₄N₈NaO₁₃S ([M+Na]⁺): 1145.4994, found: 1145.4981.

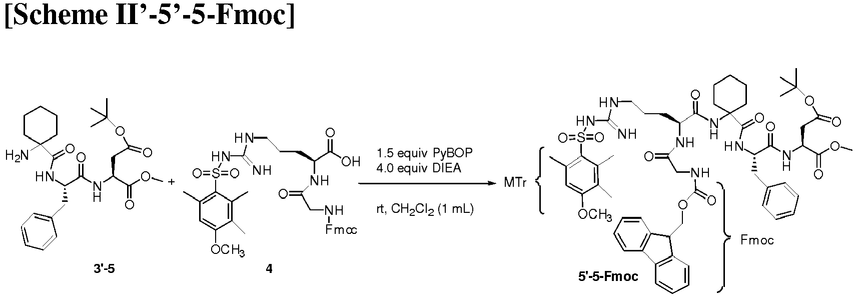

In a dry 25-mL, two-necked, round-bottomed flask was charged with coupling reagent (1.5 equiv) in DCM (1 mL/mmol) and treated under stirring with DIEA (4.0 equiv) at 0 °C for 5 min. Fmoc-Gly-Arg(Mtr)-OH (4) (149.6 mg, 0.22 mmol) was added at 0 °C for 20 min, and mixed with tripeptide (**3'-5**) (106.8 mg, 0.22 mmol). The ice bath was removed after 20 min and the stirring continued at room temperature 10 days. The crude product was dried in vacuo and purified by column chromatography on silica gel to obtain Fmoc-protected peptide (**5'-5-Fmoc**).
Yield: 30% (PyBOP); 65% (by MoO₂Cl₂/4-nitrobenzoic anhydride).
Purified by column chromatography (EtOAc/MeOH = 99.5:0.5, R*_{f}*=0.6); ¹H NMR (400 MHz, CDCl₃) δ 7.76 (d, *J* = 8.0, 1H, NH), 7.70 (d, *J* = 8.2, 2H), 7.56 (d, *J* = 6.4, 1H, NH), 7.51 (d, *J* = 7.2, 2H), 7.42 (bd, 1H, NH), 7.38 (d, *J* = 8.0, 2H), 7.36-7.16 (m, 2H), 7.11-7.06 (m, 5H), 6.86 (d, *J* = 7.6 Hz, 1H, NH), 6.50 (s, 1H), 6.38 (bs, 2H), 4.84 (dd, *J* = 14.0, 6.6 Hz, 1H), 4.67 (dd, *J* = 13.8, 8.0 Hz, 1H), 4.45-4.27 (m, 2H), 4.20-4.11 (m, 1H), 3.90-3.89 (m, 1H), 3.79 (s, 3H, OCH₃), 3.73-3.71 (m, 1H), 3.65 (s, 3H, CO₂CH₃), 3.54-3.46 (m, 1H), 3.43-3.36 (m, 2H), 3.20-3.16 (m, 1H), 3.05 (dd, *J* = 14.0, 9.3 Hz, 1H), 2.71 (t, *J* = 8.0 Hz, 1H), 2.71 (m, 1H), 2.67 (s, 3H, CH₃), 2.61 (s, 3H, CH₃), 2.10 (s, 3H, CH₃), 2.01-1.79 (m, 3H), 1.76-1.51 (m, 2H), 1.50-1.41 (m, 2H), 1.39 (s, 9H, C(CH₃)₃), 1.32-1.02 (m, 6H), 0.94-0.82 (m, 2H); HRMS (ESI) calcd for C₅₈H₇₄N₈O₁₃S (M+H⁺): 1122.5098; found: 1122.5096.

### Example 4

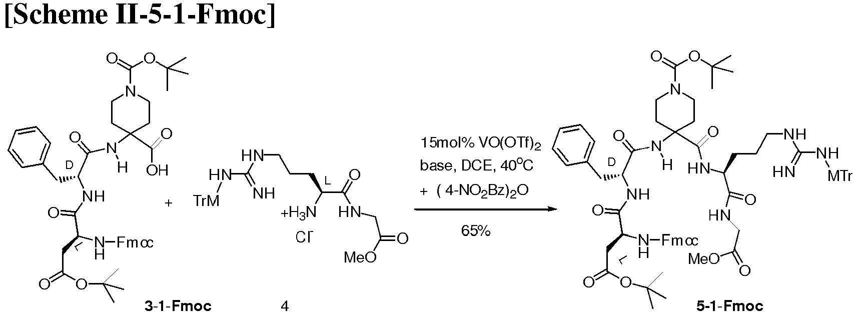

Similar procedure like the one for [Scheme II-5-5-Fmoc] provide the product in 65% yield: Purified by column chromatography (EtOAc/MeOH = 9.5:0.5, R*_{f}*=0.40); ¹H NMR (400 MHz, CDCl₃): δ 7.76 (d, 2 H, *J* = 7.4 Hz), 7.59-7.56 (m, 2 H), 7.38 (t, 2 H, *J* = 7.6 Hz), 7.28-7.15 (m, 8 H), 6.64 (br, 1 H), 6.58 (br, 1 H), 4.69 (br, 1 H), 4.55-4.45 (m, 1H), 4.40 (dd, 1 H, *J* = 7.2, 6.4 Hz), 4.38-4.24 (m, 2H), 4.20 (t, 1 H, *J* = 7.2 Hz), 4.16-4.08 (m, 2H), 3.82 (s, 3H, OCH₃), 3.76-3.71 (m, 2H), 3.66-3.62 (m, 1H), 3.56 (s, 3H, CO₂CH₃), 3.44-3.40 (m, 1H), 3.34-3.30 (m, 2H), 3.18-3.12 (m, 2H), 3.01-2.93 (m, 1H), 2.66 (s, 3H), 2.57 (s, 3H), 2.27-2.15 (m, 2H), 2.09 (s, 3H), 2.25-2.04 (m, 4H), 1.78-1.60 (m, 2H), 1.56-1.52 (m, 2H), 1.40 (s, 9H, C(CH₃)₃), 1.36 (s, 9H, C(CH₃)₃); HRMS (ESI), calculated for C₆₂H₈₂N₉O₁₅S ([M+H]⁺): 1224.5651, found: 1224.5654.

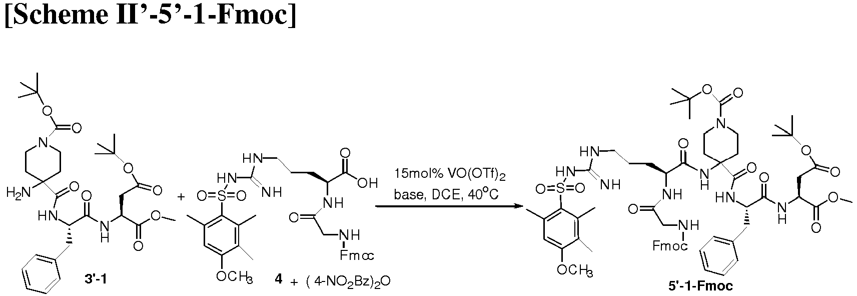

Similar procedure like the one for [Scheme II'-5'-5-Fmoc] provide the product in 72% yield: Purified by column chromatography (EtOAc/MeOH = 9.5:0.5, R*_{f}*=0.4); ¹H NMR (400 MHz, CDCl₃) δ 7.62 (d, *J* = 8.2, 1H, NH), 7.60 (d, *J* = 8.0, 2H), 7.53 (d, *J* = 6.4, 1H), 7.45 (d, *J* = 7.2, 2H), 7.30 (bd, 1H, NH), 7.36 (d, *J* = 8.0, 2H), 7.34-7.18 (m, 6H), 7.18-7.10 (m, 5H), 6.74 (d, *J* = 7.6 Hz, 1H, NH), 6.40 (s, 1H), 6.25 (bs, 2H), 4.81 (dd, *J* = 13.8, 6.8 Hz, 1H), 4.70 (dd, *J* = 13.8, 8.0 Hz, 1H), 4.52-4.31 (m, 3H), 4.18-4.08 (m, 1H), 3.95-3.86 (m, 2H), 3.77 (s, 3H, OCH₃), 3.73-3.71 (m, 1H), 3.62 (s, 3H, OCH₃), 3.68-3.60 (m, 2H), 3.57-3.46 (m, 1H), 3.48-3.36 (m, 2H), 3.18-3.12 (m, 1H), 3.08 (t, *J* = 8.0 Hz, 1H), 2.71 (dd, *J* = 14.0, 9.3 Hz, 1H), 2.73-2.67 (m, 1H), 2.63 (s, 3H, CH₃), 2. 55 (s, 3H, CH₃), 2.11 (s, 3H, CH₃), 2.11-1.83 (m, 6H), 1.76-1.61 (m, 8H), 1.55-1.45 (m, 6H), 1.36 (s, 9H, C(CH₃)₃), 1.36-1.06 (m, 8H), 0.92-0.84 (m, 2H); HRMS (ESI) calcd for C₆₂H₈₂N₉O₁₅S (M+H⁺): 1224.5651; found: 1224.5662.

### Example 5

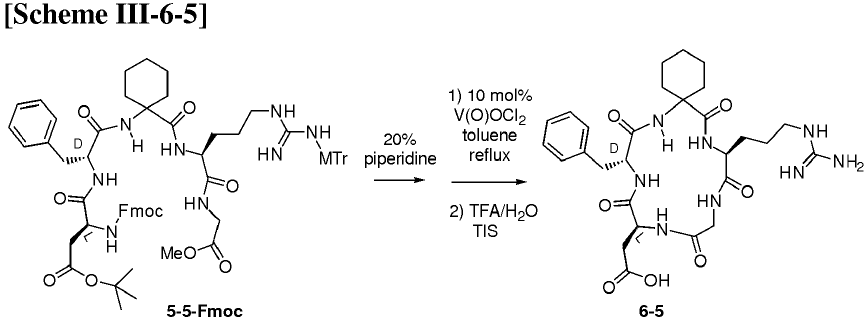

After Fmoc deprotection and cyclization of 5-5-Fmoc:

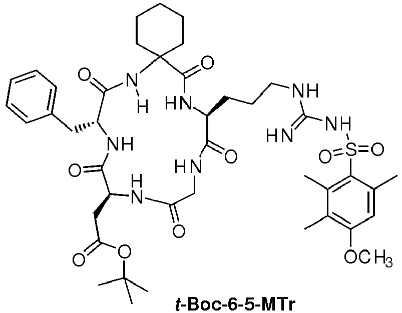

Data for ***t*-Boc-6-5-MTr:** ¹H NMR (400 MHz, CDCl₃): δ 8.00 (s, 1H, C=NH), 7.88 (bs, 1H, NH), 7.75 (bs, 1H, NH), 7.64 (bs, 1H, NH), 7.42-7.13 (m, 6H), 7.01 (br, 1H, NH), 6.52 (br, 1H, MTR-H), 6.27 (br, 2H, NH), 4.79-4.71 (m, 1H), 4.62-4.51 (m, 1H), 4.31-4.22 (m, 1H), 4.09-4.02 (m, 2H), 3.81 (s, 3H, OCH₃), 3.61-3.56 (m, 1H), 3.54-3.25 (m, 2H, CH₂-quanidine), 3.21-3.09 (m, 1H), 3.03-2.87 (m, 2H), 2.69 (s, 3H, CH₃), 2.59 (s, 3H, CH₃), 2.59-2.47 (m, 1H), 2.11 (s, 3H, CH₃), 2.01-1.68 (m, 5H), 1.59-1.52 (m, 4H), 1.52-1.38 (m, 4H), 1.52 (s, 9H, C(CH₃)₃), 0.86 (quin, 2H, *J* = 7.8 Hz); HRMS (ESI), calculated for C₄₂H₆₁N₈O₁₀S ([M+H]⁺): 869.4231, found: 869.4201; TLC: R*_{f}* 0.42 (EtOAc/MeOH, 3/1).

After MTR, and t-Boc deprotection of ***t*-Boc-6-5-MTr:**
Data for Compound **6-5:** ¹H NMR (400 MHz, CDCl₃) δ 10.02 (bs, 2H, G-NH₂⁺), 8.25 (bs, 1H, G-NH), 7.83 (bd, *J* = 8.2,1H, amide NH), 7.75 (d, *J* = 8.0,1H, amide NH), 7.71 (d, *J* = 8.4,1H, amide NH), 7.62 (t, *J* = 8.0, 1H, amide NH), 7.36 (d, *J* = 8.2,1H, amide NH), 7.22-7.11 (m, 5H, Ph), 4.72 (dd, *J* = 15.8, 7.8, 1H), 4.55 (bt, 1H), 4.36 (bs, 2H), 4.20 (dd, *J* = 16.0, 7.2, 1H), 3.36 (t, *J* = 14.8 Hz, 2H), 3.25-3.12 (m, 4H), 2.64 (dd, *J* = 7.6, 16.0 Hz, 1H), 2.57 (dd, *J* = 16.0, 10.4, 1H), 1.84-1.72 (m, 4H), 1.58-1.50 (m, 4H), 1.48-1.38 (m, 4H); HRMS (ESI) calcd for C₂₈H₄₁N₈O₇ (M⁺+H): 601.3093; found: 601.3094; HPLC analysis: (C18, 250x4.6mm, 0.5 (mL/min), λ = 254 nm). a.1%TFA in H₂O/ACN (95:5) 30 min; b.1%TFA in H₂O/ACN (5:95) 31-60min; *t*_{R} 36.71, 46.17 min.

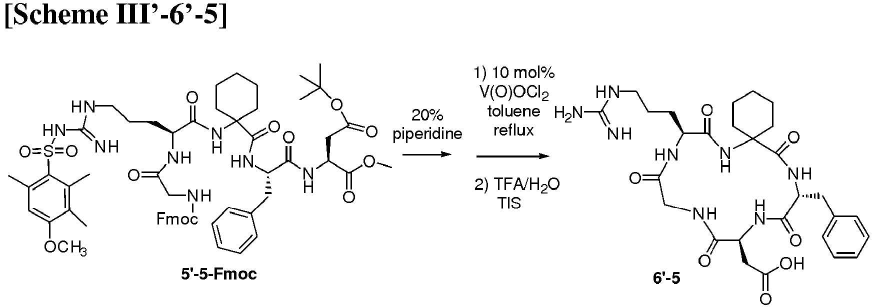

A solution of 40.5 mg (0.036 mmol) of the Fmoc-protected peptide (**5'-5-Fmoc**) was treated with 20% piperidine in DCM (1 mL) for 1 hour at room temperature. After removal of piperidine by coevaporation with methanol, the crude product was dried in vacuo and purified by column chromatography on silica gel. The resulting product was subjected to intramolecular amide bond formation by treatment with 10 mol% VOOCl₂ V(O)(acac)₂, or Ti(O)(acac)₂ in refluxed toluene for 18 h. The resulting crude mixture was cooled to ambient temperature and concentrated. The crude residue was dissolved in trifluoroacetic acid (5 mL) and H₂O (1 mL) and then treated with thioanisole (1 mL). The mixture was induced precipitation with di-isopropyl ether (5 mL) and the solid washed with di-isopropyl ether and dried in vacuo to obtain the cyclopeptide (**6'-5**). The cyclic pentapeptide can be further purified by HPLC on a reverse phase C-18 column (gradient: 95/5 to 80/20, H₂O/CH₃CN) to give 18 mg (69% yield) of pure **6'-5.**

After Fmoc deprotection and cyclization of **5'-5-Fmoc:**

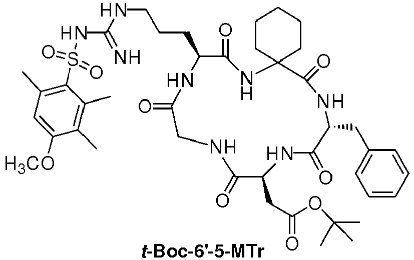

**Data for *t*-Boc-6'-5-MTr:** ¹H NMR (400 MHz, CDCl₃) δ 7.68 and 7.28 (m, 2H, imine and amide), 7.22-7.12 (m, 5H, Ph group), 6.50 (s, 1H, amide), 6.33 (br, 2H, amide), 4.80 (dd, *J* = 6.0, 6.9 Hz, 1H), 4.59-4.55 (m, 1H), 4.45-4.36 (m, 1H), 3.80 (s, 3H, OCH₃-Ph), 3.33-3.15 (m, 3H), 2.79 (t, *J* = 8.4 Hz, 1H), 2.65 (s, 3H, CH₃-Ph), 2.59 (s, 3H, CH₃-Ph), 2.31 (t, *J* = 10.4 Hz, 1H), 2.08 (s, 3H, CH₃-Ph), 2.03-1.95 (m, 4H), 1.64-1.41 (m, 5H), 1.36 (s, 9H, tBu), 1.30-1.27 (m,2H), 1.25-1.22 (m, 4H); R*_{f}* 0.5 (EtOAc/MeOH, 9/1); HRMS (ESI) calcd for C₄₂H₆₁N₈O₁₀S (M⁺+H): 869.4231; found: 869.4233.

After MTr and *tert*-Boc deprotection of ***t*-Boc-6'-5-MTr:**

Data for Compound **6'-5:** ¹H NMR (400 MHz, CDCl₃) δ 10.12 (bs, 2H, G-NH₂⁺), 8.22 (bs, 1H, G-NH), 7.79 (bd, *J* = 8.4,1H, amide NH), 7.72 (d, *J* = 8.2,1H, amide NH), 7.68 (d, *J* = 8.2,1H, amide NH),7.58 (t, *J* = 8.0, 1H, amide NH), 7.41 (d, *J* = 8.2,1H, amide NH), 7.24-7.13 (m, 5H, Ph), 4.68 (dd, *J* = 13.2, 6.8, 1H), 4.45 (bt, 1H), 4.30 (bs, 2H), 4.22 (dd, *J* = 16.0, 7.2, 1H), 3.30 (t, *J* = 14.0 Hz, 2H), 3.18-3.00 (m, 4H), 2.68 (dd, *J* = 7.6, 16.0 Hz, 1H), 2.55 (dd, *J* = 16.0, 10.4, 1H), 1.78-1.01 (m, 10H); HRMS (ESI) calcd for C₂₈H₄₁N₈O₇ (M⁺+H): 601.3098; found: 601.3090; HPLC analysis: (C18, 250x4.6mm, 0.5 (mL/min), λ = 254 nm). a.1%TFA in H₂O/ACN (90:10) 30min; b.1%TFA in H₂O/ACN (10:90) 31-60min; *t*_{R} 34.6, 42.6 min.

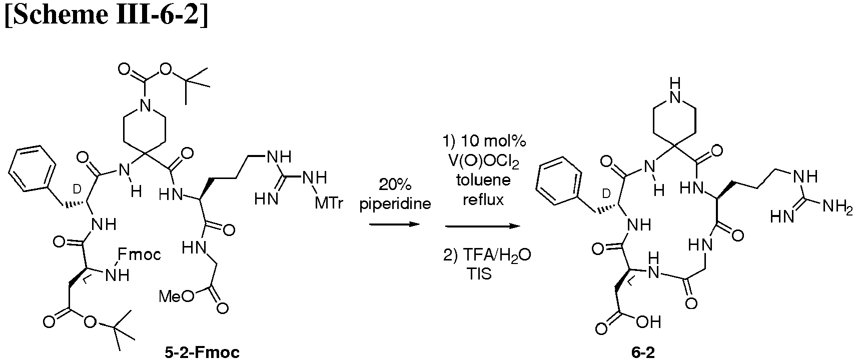

After Fmoc deprotection and cyclization of **5-2-Fmoc:**

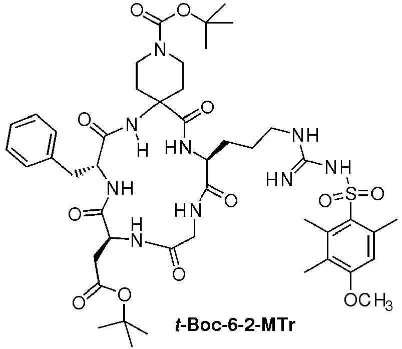

Data for ***t*-Boc-6-2-MTr:** ¹H NMR (400 MHz, CDCl₃): δ 7.98 (s, 1H, C=NH), 7.86 (bs, 1H, NH),7.62 (bs, 1H, NH), 7.38-7.11 (m, 6H), 7.00 (br, 1H), 6.58 (br, 1H, NH), 6.30 (br, 1H, NH), 4.80-4.74 (m, 1H), 4.65-4.57 (m, 1H), 4.34-4.26 (m, 1H), 4.10-4.07 (m, 2H), 3.71-3.67 (m, 2H), 3.60-3.54 (m, 4H), 3.52-3.31 (m, 2H, CH₂-quanidine), 3.48 (s, 3H, OCH₃), 3.18-3.09 (m, 1H), 3.05-2.91 (m, 2H), 2.69 (s, 3H, CH₃), 2.59 (s, 3H, CH₃), 2.11 (s, 3H, CH₃), 2.21-1.78 (m, 5H), 1.63-1.57 (m, 2H), 1.55 (s, 9H, C(CH₃)₃), 1.53-1.43 (m, 2H), 1.50 (s, 9H, C(CH₃)₃); HRMS (ESI), calculated for C₄₆H₆₈N₉O₁₂S ([M+H]⁺): 970.4708, found: 970.4715; TLC: R*_{f}* 0.33 (EtOAc/MeOH, 3/1).

After MTr and *tert*-Boc deprotection of ***t*-Boc-6-2-MTr:**
Data for Compound **6-2:** ¹H NMR (400 MHz, CDCl₃): 9.40 (bs, 2H, G-NH₂⁺), 8.55 (bs, 1H, G-NH), 7.63 (bd, *J* = 8.2, 1H, amide NH), 7.69 (d, *J* = 8.0, 1H, amide NH), 7.63 (d, *J* = 8.4, 1H, amide NH), 7.55 (t, *J* = 8.0, 1H, amide NH), 7.38 (d, *J* = 8.2,1H, amide NH), 7.24-7.14 (m, 5H, Ph), 6.81 (bs, 2H, G-NH₂), 4.82 (dd, *J* = 15.8, 7.8, 1H), 4.70 (bt, 1H), 4.40 (bs, 2H), 4.15 (dd, *J* = 16.0, 7.2, 1H), 3.40 (t, *J* = 14.8 Hz, 2H), 3.22-3.12 (m, 4H), 2.97-2.88 (m, 2H), 2.81-2.72 (dd, *J* = 7.6, 16.0 Hz, 1H), 2.68-2.62 (m, 2H), 2.60 (dd, *J* = 16.0, 10.4, 1H), 2.24-2.06 (m, 3H), 1.84-1.77 (m, 4H), 1.54-1.48 (m, 2H); HRMS (ESI) calcd for C₂₇H₄₀N₉O₇ (M⁺+H): 602.3045; found: 602.3041; HPLC analysis: (C18, 250x4.6mm, 0.5 (mL/min), λ = 254 nm). a.1%TFA in H₂O/ACN (90:10) 30 min; b.1%TFA in H₂O/ACN (5:95) 31-60 min; *t*_{R} 42.5, 51.2 min.

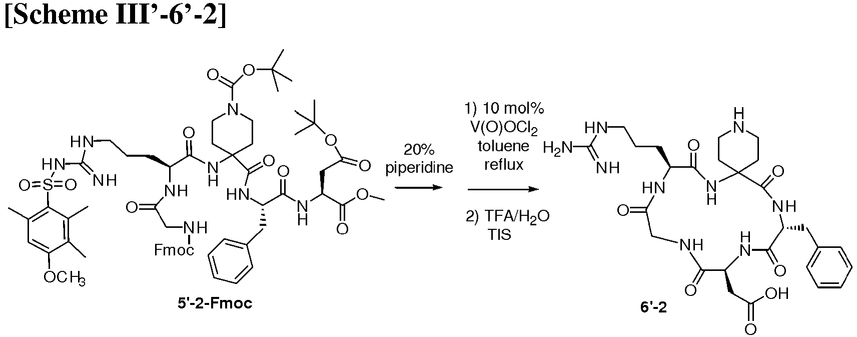

After Fmoc, MTR, and t-Boc deprotection of **5'2-Fmoc:**
Data for Compound **6'-2:** ¹H NMR (400 MHz, CDCl₃) δ 9.68 (bs, 2H, G-NH₂⁺), 8.09 (bs, 1H, G-NH), 7.68 (bd, *J* = 8.4,1H, amide NH), 7.64 (d, *J* = 8.2,1H, amide NH), 7.60 (d, *J* = 8.2,1H, amide NH), 7.50 (t, *J* = 8.0, 1H, amide NH), 7.45 (d, *J* = 8.2,1H, amide NH), 7.27-7.16 (m, 5H, Ph), 4.65 (dd, *J* = 15.8, 7.6, 1H), 4.72 (bt, 1H), 4.27 (bs, 2H), 4.15 (dd, *J* = 15.8, 7.4, 1H), 3.32 (t, *J* = 15.2 Hz, 2H), 3.24-3.15 (m, 4H), 2.80-2.65 (m, 4H), 2.64 (dd, *J* = 7.6,16.0 Hz, 1H), 2.58 (dd, J= 16.0,10.4, 1H), 2.14-2.06 (m, 2H), 2.05 (bs, 1H, NH), 1.89-1.82 (m, 2H), 1.78-1.72 (m, 4H), 1.67-1.48 (m, 2H); HRMS (ESI) calcd for C₂₇H₄₀N₉O₇ (M⁺+H): 602.3045; found: 602.3049; HPLC analysis: (C18, 250x4.6mm, 0.5 (mL/min), λ = 254 nm). a.1%TFA in H₂O/ACN (90:10) 30 min; b.1%TFA in H₂O/ACN (5:95) 31-60 min; *t*_{R} 41.8, 50.4 min.

## Claims

1. A cyclopeptide represented by the following formula (I-1) or (I-3):

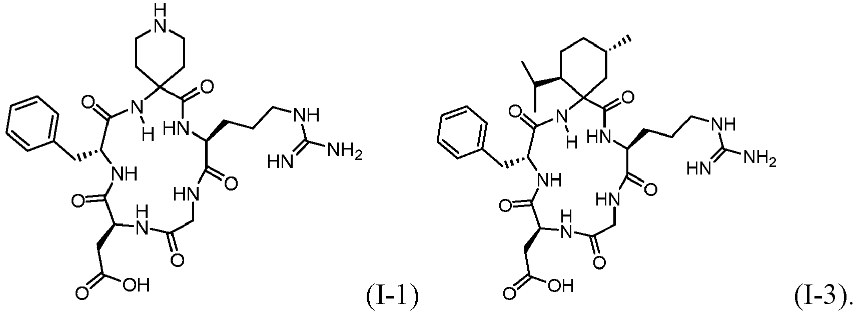

2. A pharmaceutical or cosmetic composition, comprising:
an excipient, a Cu (II) ion or a VO(II) ion; and
a cyclopeptide represented by the following formula (I-1) or (I-3):

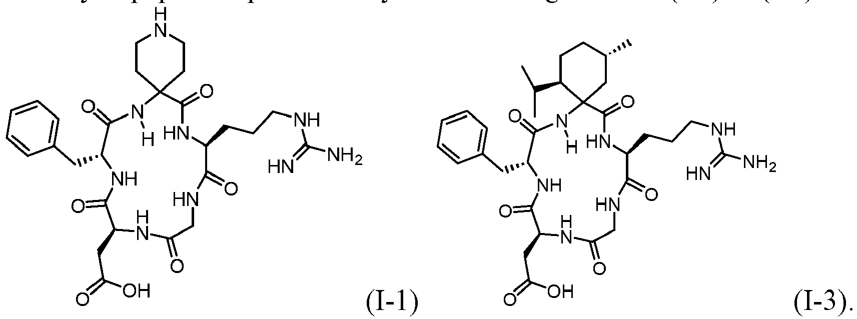

## Patentansprüche

1. Ein Cyclopeptid gemäß einer der folgenden Formeln (I-1) oder (I-3):

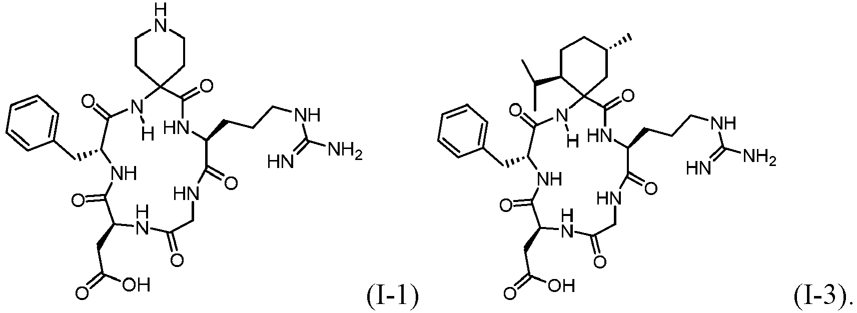

2. Eine pharmazeutische oder kosmetische Zusammensetzung aufweisend:
einen Hilfsstoff, ein Cu(II) Ion oder ein VO(II) Ion; und
ein Cyclopeptid gemäß einer der folgenden Formeln (I-1) oder (I-3):

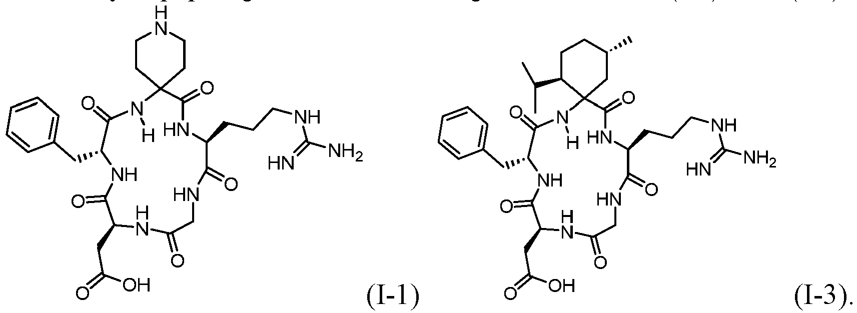

## Revendications

1. Cyclopeptide représenté par la formule suivante (I-1) ou (I-3) :

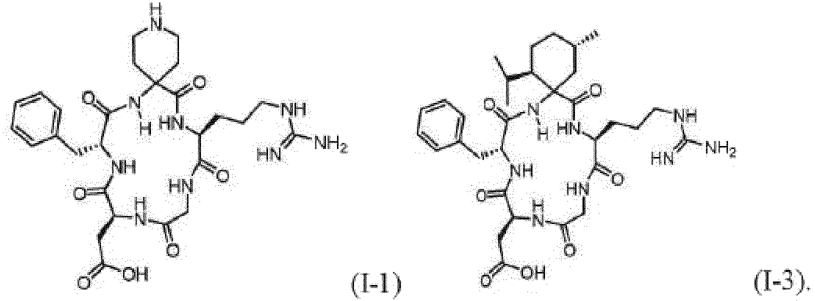

2. Composition pharmaceutique ou cosmétique, comprenant :
un excipient, un ion Cu (II) ou un ion VO(II) ; et
un cyclopeptide représenté par la formule suivante (I-1) ou (I-3) :
